# EUROPEAN PATENT APPLICATION

(11) **EP 4 851 066 A1**
(43) Date of publication of application: **09.12.2026**
(21) Application number: 25748503.7
(22) Date of filing: 07.01.2025
(51) Int. Cl.: B65D 75/30, A61K 8/02, A61Q 19/00, B32B 3/30, B32B 5/02, B32B 27/10, B65D 65/40, D21H 19/10, D21H 27/10, D21H 27/30

(54) **BACKING PAPER FOR SKIN PACK AND SKIN PACK PACKAGING**

(30) Priority: 29.01.2024 JP 2024011090
(71) Applicant: Oji Holdings Corporation, Chuo-ku Tokyo 104-0061 (JP)
(72) Inventor: NISHIDA, Haruka, Tokyo 104-0061 (JP); HIRANO, Hironobu, Tokyo 104-0061 (JP); ISHIKAWA, Yumiko, Tokyo 104-0061 (JP)
(74) Representative: Novagraaf International SA
(86) International application number: PCT/JP2025/000191
(87) International publication number: WO 2025/164212

(57) **Abstract**

A paper board for skin pack, including a thermoplastic resin layer, a paper substrate, and a coating layer in the listed order, in which a degree of fibrillation of pulp in the paper substrate is 1.0% or less, the coating layer contains a resin, a surface roughness of a surface of the coating layer, the surface being located opposite to the paper substrate, is 1.50 to 4.20 µm, and a geometric mean of a specific tensile strength in a longitudinal direction and a specific tensile strength in a lateral direction of the paper board for skin pack is 37.0 N·m/g or more.

## Description

### TECHNICAL FIELD

The present disclosure relates to a paper board for skin pack, and a skin pack package.

### BACKGROUND ART

Skin pack packages in which trays and food products placed thereon are vacuum-packed with barrier films have been progressively used in the field of food packaging, in order to realize freshness retention and lifetime elongation of food products. Such skin pack packages, when used particularly in packaging of dressed meat or the like, can be inhibited from causing dripping (moisture leaked from food products during preservation), and thus is advantageous also from the viewpoint of allowing for best-before date extension and food waste reduction.

In this situation, there have also been developed skin pack packages in which foods are placed on paper boards with paper substrates, instead of trays of resins such as polystyrene and polypropylene, and packed with films, in consideration of, for example, designability with printability and reduction of environmental load. In production of such skin pack packages, paper dust occurs during application of punching processing to paper boards or during application of friction to paper board backsides due to conveyance or the like of paper boards, thereby causing the problems of foreign matter inclusion, print dropouts, and the like.

There has been then developed a paper board for skin pack, in which paper dust is less generated. For example, Patent Document 1 describes a paper board for skin pack, in which generation of paper dust can be suppressed by setting the taper stiffness in the longitudinal direction to 150 mN·m or less and setting the taper stiffness in the lateral direction to 80 mN·m or less.

### PRIOR ART DOCUMENTS

### PATENT DOCUMENTS

Patent Document 1: JP 2021-191690 A

### SUMMARY OF THE INVENTION

### TECHNICAL PROBLEM

A skin pack package can include the content fixed at the center of a paper board, and therefore many products can be preserved or displayed in the state of being suspended or placed on a slope. The mode of use of such a skin pack package is thus diversified, and a paper board for skin pack is demanded to include properties suited for the mode of use. For example, such a skin pack package, when preserved or displayed in the state of being placed on a slope, is demanded to hardly slide when placed on the slope. In this regard, a paper board for skin pack is, of course, required to also include properties demanded in use for skin pack packaging, for example, no warpage due to film shrinkage during skin pack packaging, and such properties cannot be sacrificed for impartment of other additional properties.

In view of such circumstances, depending on the mode of use of a skin pack package, there is a need for development of a new technique for suppressing generation of paper dust, separate from the technique described in Patent Document 1, in order to impart properties suited for the mode of use to a paper board for skin pack.

An object of the present disclosure is to provide a paper board for skin pack, which causes less generation of paper dust, which hardly slides when placed on a slope, and which is also inhibited from warping during skin pack packaging.

### SOLUTION TO PROBLEM

The inventors of the present disclosure have made intensive studies, and as a result, have found that the above problem can be solved by allowing the specific tensile strength of a paper board for skin pack and the degree of fibrillation of pulp in a paper substrate to fall within predetermined ranges and providing a coating layer having a predetermined surface roughness on one surface of the paper substrate. Specifically, the gist of the present disclosure is as follows.
[1] A paper board for skin pack, comprising a thermoplastic resin layer, a paper substrate, and a coating layer in the listed order, wherein
   a degree of fibrillation of pulp in the paper substrate is 1.0% or less,
   the coating layer contains a resin,
   a surface roughness of a surface of the coating layer, the surface being located opposite to the paper substrate, is 1.50 to 4.20 µm, and
   a geometric mean of a specific tensile strength in a longitudinal direction and a specific tensile strength in a lateral direction of the paper board for skin pack is 37.0 N·m/g or more.
[2] The paper board for skin pack according to [1], wherein a mass per unit area of the resin in the coating layer is 0.10 to 1.90 g/m².
[3] The paper board for skin pack according to [1] or [2], wherein the resin contained in the coating layer contains one or more selected from the group consisting of a water-soluble resin and a water-suspendable resin (preferably starch).
[4] The paper board for skin pack according to [3], wherein the water-soluble resin contains one or more selected from the group consisting of oxidized starch, cationized starch, polyvinyl alcohol, and polyacrylamide (preferably one or more selected from the group consisting of oxidized starch and cationized starch).
[5] The paper board for skin pack according to any of [1] to [4], wherein the geometric mean of a specific tensile strength in a longitudinal direction and a specific tensile strength in a lateral direction of the paper board for skin pack is 53.0 N·m/g or less.
[6] The paper board for skin pack according to any of [1] to [5], wherein the paper substrate contains 0.10% by mass or more of a paper strengthening agent based on a total amount of the paper substrate.
[7] The paper board for skin pack according to any of [1] to [6], wherein the paper substrate is a combined sheet having a paper layer A, an adhesive layer and a paper layer B in the listed order, or a single sheet.
[8] A skin pack package comprising the paper board for skin pack according to any of [1] to [7], a contained article, and a resin film, wherein
   the contained article is contained between the thermoplastic resin layer of the paper board for skin pack and the resin film.

### ADVANTAGEOUS EFFECTS OF INVENTION

According to the present disclosure, it is possible to provide a paper board for skin pack, which causes less generation of paper dust, which hardly slides when placed on a slope, and which is also inhibited from warping during skin pack packaging.

### BRIEF DESCRIPTION OF THE DRAWINGS

[FIG. 1] The figure shows a schematic view illustrating one example of a paper board for skin pack according to the present embodiment.
[FIG. 2] The figure shows a schematic view of pulp for explaining the degree of fibrillation.

### DETAILED DESCRIPTION OF THE INVENTION

In the present disclosure, each of the designations "X or more and Y or less" and "X to Y", representing a numerical value range, means a numerical value range including the lower limit and the upper limit being end points, unless especially noted. In a case where the lower limit value and the upper limit value of a numerical value range are separately described, the numerical value range can be a combination of any lower limit value and any upper limit value.

In the present disclosure, the designation like "one or more selected from the group consisting of X, Y and Z" means any of X, Y, Z, a combination of X and Y, a combination of X and Z, a combination of Y and Z, or a combination of X, Y, and Z. In the present disclosure, the designation like "X such as X1, X2 and X3" exemplifies X1, X2, X3, and the like, as one example of X, and it is not meant that X is limited to X1, X2 and X3.

In the present disclosure, the "longitudinal direction" of each of the paper board for skin pack and the paper substrate means a direction which is a papermaking direction (MD) on the paper substrate and which is a direction in which fibers are oriented. The "lateral direction" means a direction (CD) perpendicular to the papermaking direction.

### 1. Paper board for skin pack

A first embodiment of the present disclosure relates to a paper board for skin pack (hereinafter, sometimes simply referred to as "paper board".), including a thermoplastic resin layer, a paper substrate, and a coating layer in the listed order, wherein the degree of fibrillation of pulp in the paper substrate is 1.0% or less, the coating layer contains a resin, the surface roughness of a surface of the coating layer, the surface being located opposite to the paper substrate, is 1.50 to 4.20 µm, and the geometric mean of the specific tensile strength in the longitudinal direction and the specific tensile strength in the lateral direction of the paper board for skin pack is 37.0 N·m/g or more. The paper board for skin pack according to the present embodiment causes less generation of paper dust, hardly slides down when placed on a slope, and also hardly warps upward during skin pack packaging. Therefore, a skin pack package in which this paper board for skin pack is used is suited to be preserved, displayed, and/or the like in a state of being placed on a slope.

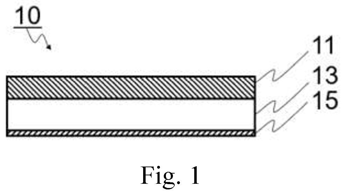

FIG. 1 illustrates one aspect of the paper board for skin pack according to the present embodiment. In FIG. 1, a paper board 10 for skin pack includes a thermoplastic resin layer 11, a paper substrate 13, and a coating layer 15 in the listed order. The paper board 10 for skin pack may include any other layer than these, for example, any other layer described below (not illustrated).

The reason why it is possible in the present embodiment to not only achieve suppression of sliding-down of the paper board placed on a slope and upward warpage of the paper board during skin pack packaging, but also suppress generation of paper dust from the paper board is presumed as follows.

First, it is considered that, when the coating layer is provided on one surface of the paper substrate, a surface of the paper substrate is covered to reduce exposure of pulp, thereby causing less generation of paper dust.

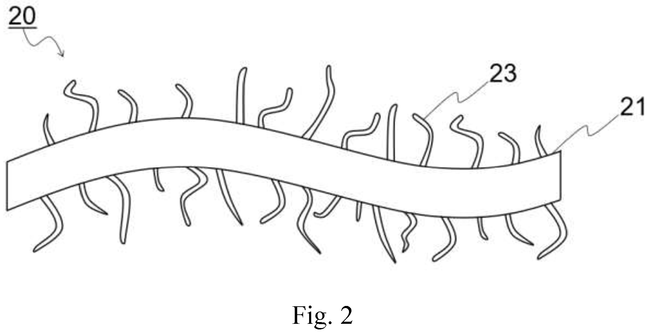

In the present embodiment, the paper substrate used here is constituted by pulp having a low degree of fibrillation. The degree of fibrillation is also called "degree of external fibrillation". As described with reference to FIG. 2, the degree of fibrillation is an index indicating how many fuzzy external fibrils (fine fibers) 23 are present on a surface of a fibril 21 within fiber 20. Such external fibrils drop from the fibrils due to punching processing, friction, and the like of the paper board, and serve as paper dust. Therefore, it is considered that generation of paper dust can be suppressed by use of a paper substrate formed with pulp having a low degree of fibrillation.

The degree of fibrillation is here calculated by expression (1), based on the description by H. R. Motamedian et. al., "Mechanisms of strength and stiffness improvement of paper after PFI refining with a focus on the effect of fines", Cellulose (2019) 26: 4099-4124.
[Expression 1] $The degree of fibrillation \left(%\right) = \frac{{A}_{Exf}}{{A}_{F} + {A}_{Exf}} \times 100$ (1)
A_{F}: Area of fibril
A_{Exf}: Total area of external fibrils
A_{F} and A_{Exf} are areas measured by projection of the fibers from above.

When skin pack packaging is performed, the contained article is disposed on the thermoplastic resin layer of the paper board for skin pack. A surface of the paper substrate, the surface being located opposite to a surface of the paper substrate, the surface being in contact with the thermoplastic resin layer, is still exposed without being covered with any packaging film even after skin pack packaging. Therefore, if the coating layer is provided on a surface of the paper substrate, the surface being located opposite to a surface of the paper substrate, the surface being in contact with the thermoplastic resin layer, generation of paper dust can be suppressed as described above, but fine irregularities on a paper substrate surface are covered with the coating layer to reduce the surface roughness, and thus a problem is that the paper board easily slides down when the skin pack package is preserved, displayed, and/or the like in a state of being placed on a slope. On the other hand, it is considered that, when the degree of fibrillation of pulp is small, the void between pulp fibers is large and irregularities are easily made on a paper substrate surface, thereby resulting in an increase in surface roughness of the paper substrate itself, so that the surface roughness can be ensured at a certain level due to surface irregularities of the paper substrate even if the coating layer is provided, and the paper board can hardly slide even in a case where the skin pack package is preserved, displayed, and/or the like in a state of being placed on a slope.

Thus, pulp small in degree of fibrillation can be used to suppress generation of paper dust from the paper board and allow the paper board to hardly slide. On the other hand, the external fibrils also have the effect of increasing the surface area of the fiber and increasing the binding area between the fibers, thereby strengthening binding between the fibers to enhance the specific tensile strength, and therefore a small degree of fibrillation of pulp leads to a decrease in specific tensile strength of the paper board for skin pack. In a case where the specific tensile strength of the paper board for skin pack is excessively low, an edge of the paper board warps upward due to thermal shrinkage of a packaging film during skin pack packaging, resulting in deterioration in visual quality of the skin pack package. Therefore, in the present embodiment, upward warpage of the paper board during skin pack packaging can be suppressed by not only keeping the degree of fibrillation of pulp at a predetermined value or less, but also allowing the paper substrate to contain, for example, a paper strengthening agent, thereby enhancing the specific tensile strength of the paper substrate and imparting the specific tensile strength that can be resistant to shrinkage force of a packaging film, to the paper board for skin pack. In addition, the specific tensile strength of the paper substrate is enhanced, in other words, the number of bonding points between the fibers is increased and the binding force between the fibers is increased. Accordingly, it is considered that the specific tensile strength of the paper board for skin pack can be set to a predetermined value or more to also inhibit the external fibrils from being dropped from the paper substrate.

### (Geometric mean of the specific tensile strength in the longitudinal direction and the specific tensile strength in the lateral direction of paper board for skin pack)

The geometric mean of the specific tensile strength in the longitudinal direction and the specific tensile strength in the lateral direction of the paper board for skin pack ((Specific tensile strength in longitudinal direction × Specific tensile strength in lateral direction)^{1/2}; hereinafter, sometimes referred to as "geometric mean of specific tensile strength".) is usually 37.0 N·m/g or more, preferably 38.0 N·m/g or more, more preferably 40.0 N·m/g or more, further preferably 41.0 N·m/g or more, still more preferably 42.0 N·m/g or more, particularly preferably 45.0 N·m/g or more. When the geometric mean of specific tensile strength of the paper board for skin pack is the above lower limit or more, generation of paper dust from the paper board and upward warpage of the paper board during skin pack packaging can be suppressed.

The "specific tensile strength in the longitudinal direction" refers to a value determined by dividing the tensile strength in the longitudinal direction by the basis weight, and the "specific tensile strength in the lateral direction" refers to a value determined by dividing the tensile strength in the lateral direction by the basis weight.

The geometric mean of specific tensile strength of the paper board for skin pack is usually 56.0 N·m/g or less, preferably 53.0 N·m/g or less, more preferably 52.0 N·m/g or less, further preferably 51.0 N·m/g or less, still more preferably 50.0 N·m/g or less, particularly preferably 48.0 N·m/g or less. When the geometric mean of specific tensile strength of the paper board for skin pack is the above upper limit or less, the cushioning properties of the paper board for skin pack are enhanced and it easily absorbs impact, thereby enabling generation of folding due to drop impact to be effectively suppressed. In a case where the skin pack package is preserved, displayed, and/or the like in a state of being placed on a slope or being suspended, the skin pack package has an increased risk of dropping when operated, and the skin pack package, if drops, may cause generation of folding of the paper board for skin pack due to drop impact. Therefore, the paper board for skin pack is demanded to have the property of hardly folding due to drop impact in order that the skin pack package is preserved, displayed, and/or the like in a state of being placed on a slope or being suspended. Accordingly, the geometric mean of specific tensile strength of the paper board for skin pack is yet preferably the above upper limit or less because the skin pack package can be suitably preserved, displayed, and/or the like in a state of not only being placed on a slope, but also being suspended.

From the foregoing, examples of the range of the specific tensile strength of the paper board for skin pack include ranges of 37.0 to 56.0 N·m/g, 38.0 to 53.0 N·m/g, 40.0 to 52.0 N·m/g, 41.0 to 51.0 N·m/g, 42.0 to 50.0 N·m/g, and 45.0 to 48.0 N·m/g.

In the present disclosure, the "folding" of the paper board for skin pack means that any visually distinguishable fold is formed in the paper board for skin pack, due to drop impact. Accordingly, whether or not any fold is formed in the paper board is determined by visual observation, as shown in Examples below.

The method for adjusting the specific tensile strength of the paper board for skin pack is not particularly limited, and examples thereof include a method involving allowing the paper substrate to contain a predetermined amount of a paper strengthening agent. The paper strengthening agent allows the effect of increasing the bonding points between the fibers and thus increasing the specific tensile strength of the paper substrate to be obtained. Alternatively, the specific tensile strength of the paper substrate can also be adjusted by a method involving allowing the pulp constituting the paper substrate to contain a predetermined amount of needle kraft pulp (NKP). The needle kraft pulp (NKP) has a long fiber and has the effect of increasing the specific tensile strength of the paper substrate. The specific tensile strength of the paper substrate can be adjusted by such a method, thereby allowing for adjustment of the specific tensile strength of the paper board for skin pack.

The specific tensile strength in the longitudinal direction and that in the lateral direction of the paper board for skin pack are measured with a horizontal tensile tester ("CODE SE-06" manufactured by L&W), according to JIS P 8113:2006 ("Paper and Board-Determination of tensile properties-Part 2: Constant rate of elongation method", published on December 20, 2006). The geometric mean of specific tensile strength of the paper board for skin pack is calculated from the specific tensile strength in the longitudinal direction and that in the lateral direction based on the above expression.

### (Thickness of paper board for skin pack)

The thickness of the paper board for skin pack is not particularly limited, and it is preferably 450 µm or more, more preferably 500 µm or more, further preferably 550 µm or more, still more preferably 600 µm or more, and preferably 800 µm or less, more preferably 750 µm or less, further preferably 720 µm or less, still more preferably 700 µm or less. In other words, examples of the thickness of the skin pack include ranges of 450 to 800 µm, 500 to 750 µm, 550 to 720 µm, and 600 to 700 µm.

When the thickness of the paper board for skin pack is the above lower limit or more, cushioning properties are enhanced and drop impact is easily absorbed, and therefore generation of folding due to drop impact can be more effectively suppressed. When the thickness of the paper board for skin pack is the above upper limit or less, generation of folding due to drop impact can be suppressed.

The thickness of the paper board for skin pack is measured according to JIS P 8118:2014 ("Paper and Board-Determination of thickness, density and specific volume", published on November 20, 2014). The arithmetic mean value of the thicknesses at ten points of a sample is adopted as the thickness of the paper board for skin pack.

In a case where the paper board for skin pack has other layers than paper, for example, the later-described pressure-sensitive adhesive layer and thermoplastic resin layer, the thickness of each of such layers is measured based on an image of a cross section of the paper board for skin pack, observed with an electron microscope (SEM), and these measurement results are used to calculate the thickness of the paper board for skin pack. In other words, in a case where the paper board for skin pack has other layers than paper, for example, the later-described pressure-sensitive adhesive layer and thermoplastic resin layer, the thickness of the paper board for skin pack is the thickness including the thicknesses of these other layers.

### (Basis weight of paper board for skin pack)

The basis weight of the paper board for skin pack is not particularly limited, and it is preferably 450 g/m² or more, more preferably 480 g/m² or more, further preferably 500 g/m² or more, still more preferably 550 g/m² or more, and preferably 650 g/m² or less, more preferably 620 g/m² or less, further preferably 600 g/m² or less, still more preferably 600 g/m² or less. In other words, examples of the range of the basis weight of the paper board for skin pack include ranges of 450 to 650 g/m², 480 to 620 g/m², 500 to 600 g/m², and 550 to 600 g/m². When the basis weight of the paper board for skin pack falls within the above range, generation of folding due to drop impact can be suppressed.

The basis weight of the paper board for skin pack is measured according to JIS P 8124:2011 ("Paper and Board-Determination of basis weight", published on March 22, 2011).

### (Density of paper board for skin pack)

The density of the paper board for skin pack is not particularly limited, and it is preferably 0.650 g/cm³ or more, more preferably 0.700 g/cm³ or more, further preferably 0.800/cm³ or more, and preferably 1.000 g/cm³ or less, more preferably 0.950 g/cm³ or less, further preferably 0.920 g/cm³ or less. In other words, examples of the range of the density of the paper board for skin pack include ranges of 0.650 to 1.000 g/cm³, 0.700 to 0.950 g/cm³, and 0.800 to 0.920 g/cm³. When the density of the paper board for skin pack falls within the above range, generation of folding due to drop impact can be effectively suppressed.

The density of the paper board for skin pack is calculated by dividing the basis weight measured by the above method, by the thickness.

### 1-1. Paper substrate

### (Pulp)

The pulp constituting the paper substrate is not particularly limited as long as the degree of fibrillation falls within a specified range. The degree of fibrillation of the pulp in the paper substrate is usually 1.0% or less, and it is preferably 0.7% or less, more preferably 0.5% or less, further preferably 0.4% or less, still more preferably 0.3% or less, particularly preferably 0.2% or less from the viewpoint of more suppression of generation of paper dust and from the viewpoint of a more increase in surface roughness of a surface in contact with the coating layer. The lower limit of the degree of fibrillation of the pulp in the paper substrate is not particularly limited, and it is preferably 0.2% or more. In other words, examples of the range of the degree of fibrillation of the pulp in the paper substrate include ranges of 0.2 to 1.0%, 0.2 to 0.7%, 0.2 to 0.5%, 0.2 to 0.4%, and 0.2 to 0.3%.

The degree of fibrillation of the pulp can be adjusted in the above range, by the degree of beating. Beating is relieved and thus fibrillation can be suppressed to provide pulp small in degree of fibrillation. More specifically, in order to allow the pulp to have the above degree of fibrillation, beating is performed so that the Canadian standard freeness (CSF) of a pulp slurry used for production of the paper substrate is preferably 400 mL or more, more preferably 450 mL or more, further preferably 500 mL or more, still more preferably 550 mL or more, particularly preferably 600 mL or more. The upper limit of the CSF of the pulp slurry used for production of a paper layer is not particularly limited, and it is preferably 700 mL or less, more preferably 650 mL. In other words, examples of the range of the CSF of the pulp slurry, for allowing the pulp to have the above degree of fibrillation, include ranges of 400 to 700 mL, 450 to 700 mL, 500 to 650 mL, 550 to 650 mL, and 600 to 650 mL.

The type of the pulp constituting the paper substrate is not particularly limited as long as it is commonly adopted, plant-derived pulp is preferred, and wood pulp is more preferred.

Examples of the paper substrate or a paper layer described below specifically include kraft paper, high-quality paper, (white) cardboard, base paper for paper containers, milk carton base paper, cup base paper, liner paper, coated paper, one-side glazed paper, glassine paper, and graphan paper. In particular, a paper layer A and a paper layer B are each preferably paper selected from the group consisting of kraft paper, high-quality paper, (white) cardboard, base paper for paper containers, cup base paper, and one-side glazed paper, more preferably paper selected from the group consisting of high-grade cardboard, special cardboard, cup base paper, and kraft paper, in (white) cardboard, in terms of rigidity and strength. Examples of the kraft paper include bleached kraft paper, unbleached kraft paper, and one-side glazed bleached kraft paper. In particular, the kraft paper is preferably paper selected from the group consisting of bleached kraft paper and one-side glazed bleached kraft paper, in terms of printability and hygiene.

The pulp constituting the paper substrate is preferably wood pulp, more preferably kraft pulp, as described above. The kraft pulp encompasses leaf kraft pulp (LKP) and needle kraft pulp (NKP) depending on the difference in raw material. The leaf kraft pulp (LKP) is preferably leaf bleached kraft pulp (LBKP), and the needle kraft pulp (NKP) is preferably needle bleached kraft pulp (NBKP). Examples include bleached kraft pulp (BKP), unbleached kraft pulp (UKP), and oxygen bleached kraft pulp (OKP), depending on the difference in treatment state, and bleached kraft pulp (BKP) is preferred from the viewpoint of printability.

In particular, the pulp is preferably one or more selected from the group consisting of leaf kraft pulp (LKP) and needle kraft pulp (NKP), more preferably a mixture of leaf kraft pulp (LKP) and needle kraft pulp (NKP), more preferably a mixture of leaf bleached kraft pulp (LBKP) and needle bleached kraft pulp (NBKP). The mass ratio (LKP/NKP) of the leaf kraft pulp (LKP) to the needle kraft pulp (NKP) in the mixture is not particularly limited as long as it is a ratio used for common paper, and it is preferably 1/99 to 99/1, more preferably 30/70 to 70/30, further preferably 40/60 to 60/40.

In a case where the pulp is made of the leaf kraft pulp (LKP) and/or the needle kraft pulp (NKP), the content of the leaf kraft pulp (LKP) based on the total amount of the pulp is usually 0% by mass or more, preferably 1% by mass or more, more preferably 30% by mass or more, further preferably 40% by mass or more, and usually 100% by mass or less, preferably 99% by mass or less, more preferably 70% by mass or less, further preferably 60% by mass or less. In other words, examples of the content of the needle kraft pulp (NKP) based on the total amount of the pulp include ranges of 0 to 100% by mass, 1 to 99% by mass, 30 to 70% by mass, and 40 to 60% by mass.

In a case where the pulp is made of the leaf kraft pulp (LKP) and/or the needle kraft pulp (NKP), the content of the needle kraft pulp (NKP) based on the total amount of the pulp is usually 0 to 100% by mass or more, and is preferably 1% by mass or more, more preferably 30% by mass or more, further preferably 40% by mass or more, and preferably 99% by mass or less, more preferably 70% by mass or less, further preferably 60% by mass or less from the viewpoint of allowing the specific tensile strength of the paper substrate to fall within a predetermined range. In other words, examples of the content of the leaf kraft pulp (LKP) based on the total amount of the pulp include ranges of 0 to 100% by mass, 1 to 99% by mass, 30 to 70% by mass, and 40 to 60% by mass.

### (Paper strengthening agent)

The paper strengthening agent in the paper substrate is a component that increases the bonding point between the fibers to enhance the specific tensile strength. Accordingly, the specific tensile strength of the paper board for skin pack can be adjusted by adjusting the content of the paper strengthening agent in the paper substrate. The paper strengthening agent may be contained in one paper layer, or two or more paper layers, among the paper layers constituting the paper substrate. The paper strengthening agent may also be contained between such paper layers by application of the agent between such layers during papermaking with multi-layer papermaking and then combination of such layers.

Examples of the paper strengthening agent in the paper substrate include starch, cationized starch, polyacrylamide, and polyamide-aminoepichlorohydrin, and one or more selected from the group consisting of starch, cationized starch, and polyacrylamide is/are preferred.

The content of the paper strengthening agent in the paper substrate depends on the type of the paper strengthening agent, the desired specific tensile strength, and the like, and it is preferably 0.05% by mass or more, more preferably 0.10% by mass or more, further preferably 0.40% by mass or more, still more preferably 0.80% by mass or more, particularly preferably 1.00% by mass or more, and preferably 5.00% by mass or less, more preferably 4.00% by mass or less, further preferably 3.00% by mass or less, still more preferably 2.00% by mass or less, particularly preferably 1.80% by mass or less, based on the total amount of the paper substrate. In other words, examples of the range of the content of the paper strengthening agent in the paper substrate include ranges of 0.05 to 5.00% by mass, 0.10 to 4.00% by mass, 0.40 to 3.00% by mass, 0.80 to 2.00% by mass, and 1.00 to 1.80% by mass.

The content of the paper strengthening agent in the paper substrate of the paper board for skin pack is measured by mass analysis with a pyrolysis GC/MS pyrolyzer (for example, an apparatus manufactured by Agilent Technologies), of a sample obtained by punching 100 µg of all the layers of the paper substrate.

In a case where the content of the paper strengthening agent in the paper substrate is measured from the paper board for skin pack, the thermoplastic resin layer is removed by the following operation, before mass analysis. First, the paper board for skin pack is placed in a beaker in which xylene heated to 100°C is placed, and is left to stand in a state of being immersed in xylene for 30 minutes, to dissolve the thermoplastic resin. Next, the thermoplastic resin remaining on a surface of the paper substrate is wiped off with a waste cloth. In a case where the thermoplastic resin remains, the paper board is again immersed in xylene and the operation is repeated until the thermoplastic resin is completely removed.

### (Layer constitution)

The layer constitution of the paper substrate is not particularly limited as long as the geometric mean of the specific tensile strength in the longitudinal direction and the specific tensile strength in the lateral direction of the paper board falls within the above range. The paper substrate may be a combined sheet including two or more paper layers, or a single sheet composed of one paper layer, and is preferably a combined sheet having a multi-layer structure, a single sheet composed of a paper layer having a multi-layer structure with multi-layer papermaking, or a combined sheet including a paper layer having a multi-layer structure with multi-layer papermaking because the specific tensile strength of the paper board for skin pack is easily controlled.

In a case where the paper substrate is a combined sheet, the number of paper layers contained in the combined sheet, the method for layering each of the paper layers, physical properties of each of the paper layers, and the like are not particularly limited. The paper substrate preferably has a paper layer A, an adhesive layer and a paper layer B in the listed order. The combined sheet, which has a paper layer A, an adhesive layer and a paper layer B in the listed order, may be a combined sheet including only a paper layer A, an adhesive layer and paper layer B, or may be a combined sheet in which still another paper layer is layered with the adhesive layer being interposed. Here, the same paper layer as the paper layer A may be used as the paper layer B. The same paper layer as the paper layer A or the paper layer B may be used as any other paper layer.

In a case where the paper substrate is a combined sheet, the paper substrate is a layered body having a paper layer A, an adhesive layer, and a paper layer B sequentially from a surface in contact with the thermoplastic resin layer. The paper substrate may have still another paper layer as long as the effects of the present disclosure are not impaired. The materials constituting the paper layer A and the paper layer B (hereinafter, also sometimes simply referred to as "paper layer"), the layer constitution, and the like are not particularly limited.

The paper layer may be a paper layer having a single-layer structure with single-layer papermaking, or may be a paper layer having a multi-layer structure with multi-layer papermaking. The paper layer preferably has a multi-layer structure from the viewpoint of enabling the specific tensile strength to be easily controlled and from the viewpoint of enabling compounding of raw materials for each layer, the basis weight, and papermaking conditions to be arbitrarily adjusted. In a case where the paper layer has a multi-layer structure, the number of layers constituting the paper layer is usually 2 to 10, preferably 3 to 9, more preferably 4 to 8, further preferably 4 to 6.

The Oken smoothness (JIS P 8155:2010) of the paper layer is preferably 5 seconds or more, more preferably 10 to 1,000 seconds. The 75° glossiness of the paper layer is preferably 5% or more, more preferably 10 to 70% from the viewpoint of printability.

The basis weight, the thickness, and the density of the paper layer preferably fall within respective ranges so that the basis weight, the thickness, and the density of the paper board for skin pack can be adjusted within the above ranges, and specific values are as follows.

The basis weight of the paper layer A is preferably 300 to 1,000 g/m², more preferably 350 to 800 g/m², further preferably 400 to 600 g/m².

The thickness of the paper layer A is preferably 300 to 1,000 µm, more preferably 400 to 800 µm, further preferably 450 to 600 µm.

The density of the paper layer A is preferably 0.50 to 1.20 g/cm³, more preferably 0.70 to 1.00 g/cm³.

The basis weight of the paper layer B is preferably 40 to 400 g/m², more preferably 50 to 300 g/m², further preferably 60 to 150 g/m².

The thickness of the paper layer B is preferably 50 to 500 µm, more preferably 70 to 300 µm, further preferably 90 to 200 µm.

The density of the paper layer B is preferably 0.50 to 1.20 g/cm³, more preferably 0.60 to 1.00 g/cm³.

The basis weight, the density, and the thickness of the paper layer are measured respectively by the same methods as in the basis weight, the density, and the thickness of the paper board for skin pack.

The basis weight of the paper layer A and the basis weight of the paper layer B preferably satisfy a relationship of the basis weight of the paper layer A ≥ the basis weight of the paper layer B. The paper layer A is in contact with the thermoplastic resin layer and thus more hardly absorbs and desorbs moisture than the paper layer B. Therefore, the basis weight of the paper layer A is equal to or more than the basis weight of the paper layer B, and thus the paper substrate is hardly affected by moisture and can be inhibited from being curled.

In a case where the paper layer is a single sheet, the single sheet used here is preferably the above paper layer A.

### (Adhesive layer)

An adhesive layer for adhesion of the paper layer A and the paper layer B may be any one as long as it is a layer made of a material having adhesiveness, and preferably contains a thermoplastic resin. The thermoplastic resin can be used to coat at least one of the paper layers with the adhesive layer heated and melted, and attach the other of the paper layers, thereby easily providing a combined sheet.

The thermoplastic resin preferably contains one or more selected from the group consisting of a polyolefin-based resin, a polyester-based resin, polylactic acid, a styrene-based resin, and an acrylic resin, more preferably contains a polyolefin-based resin.

Examples of the polyolefin-based resin include polyethylene (low-density polyethylene (LDPE), medium-density polyethylene (MDPE), high-density polyethylene (HDPE), and linear low-density polyethylene (LLDPE), and the like), polypropylene (PP), and polymethylpentene. In particular, one or more selected from the group consisting of LDPE and MDPE is/are preferred, and MDPE is more preferred, from the viewpoints of extrusion lamination properties and ease of coating of the paper layer.

Examples of the polyester-based resin include polyethylene terephthalate and polybutylene terephthalate.

Examples of the styrene-based resin include polystyrene, acrylonitrile-styrene, and acrylonitrile-butadiene-styrene.

Examples of the acrylic resin include an acrylate polymer.

The following adhesives may be used in the adhesive layer. The adhesive is not particularly limited, and an aqueous adhesive, a solvent-based adhesive, an UV-based adhesive, and the like can be used. Among these, an aqueous adhesive is preferred as the adhesive used in the adhesive layer. The aqueous adhesive is preferably one or more selected from the group consisting of an acrylic adhesive, a polyurethane-based adhesive, and an isocyanate-based adhesive, and is more preferably an acrylic adhesive because adhesion force is easily controlled and heat resistance is excellent.

The mass per unit area (in terms of solid content) of the adhesive layer is not particularly limited, and it is preferably 1.0 to 50.0 g/m², more preferably 1.0 to 20.0 g/m². When the adhesive layer is formed, the paper layer A and/or the paper layer B is/are preferably coated with the adhesive so that the solid content is the above amount.

The adhesive layer may be formed with a single layer of a single resin, may be formed with a single layer by mixing plural resins, or may be formed in the form of plural layers including the same or different resins.

The thickness of the adhesive layer is not particularly limited, and it is preferably 1 to 100 µm, more preferably 5 to 50 µm.

### 1-2. Coating layer

The paper board for skin pack has a coating layer containing a resin. The coating layer is a layer adjacent to a surface of the paper substrate, the surface being located opposite to a surface of the paper substrate, the surface being in contact with the thermoplastic resin layer, and has the effect of suppressing generation of paper dust from the paper board.

In the present disclosure, the "coating layer" means a layer formed by coating with a coating liquid. The "coating" mentioned here is intended to encompass printing, for example, gravure printing, offset printing, flexo printing, and ink-jet printing.

The surface roughness of a surface of the coating layer, the surface being located opposite to the paper substrate, is usually 1.50 µm or more, preferably 2.00 µm or more, more preferably 2.50 µm or more, further preferably 3.00 µm or more, still more preferably 3.50 µm or more in order to ensure that the paper board for skin pack hardly slides. The surface roughness of a surface of the coating layer, the surface being located opposite to the paper substrate, is usually 4.20 µm or less, preferably 4.00 µm or less, more preferably 3.80 µm or less in order to more effectively suppress generation of paper dust from the paper board. In other words, examples of the range of the surface roughness of a surface of the coating layer, the surface being located opposite to the paper substrate, include ranges of 1.50 to 4.20 µm, 2.00 to 4.00 µm, 2.50 to 4.00 µm, 3.00 to 3.80 µm, and 3.50 to 3.80 µm.

For measurement of the surface roughness of a surface of the coating layer, the surface being located opposite to the paper substrate, first, a backside of the paper board for skin pack is photographed at a magnification of 12x with a 3D shape measuring machine ("VR3200" manufactured by Keyence Corporation). Next, the entire region photographed, in the image taken, is defined as the reference surface, wave removal at a wavelength of 5 mm or more is made with analysis software (for example, "VR3000" manufactured by Keyence Corporation), and the arithmetic mean height Sa is measured according to ISO 25178-2:2021 ("Geometrical Product Specifications (GPS)-Surface texture-Part 2: Terms, Definitions, and Surface texture parameters", published on April 2012), and then is defined as the surface roughness of a surface of the coating layer, the surface being located opposite to the paper substrate.

The resin contained in the coating layer is not particularly limited as long as the surface roughness of a surface of the coating layer, the surface being located opposite to the paper substrate, falls within the above range, and the resin preferably contains one or more selected from the group consisting of a water-soluble resin and a water-suspendable resin. Examples of the water-soluble resin include oxidized starch, cationized starch, polyvinyl alcohol, and polyacrylamide. In particular, the water-soluble resin preferably contains one or more selected from the group consisting of oxidized starch and cationized starch, more preferably contains oxidized starch, in order to more effectively suppress generation of paper dust from the paper board. Examples of the water-suspendable resin include starch, a polyvinylidene chloride resin, and polyurethane. In particular, the water-suspendable resin preferably contains starch in order to more effectively suppress generation of paper dust from the paper board.

The surface roughness of a surface of the coating layer, the surface being located opposite to the paper substrate, can be adjusted by the surface roughness of a surface of the paper substrate, the surface being in contact with the coating layer, and the mass per unit area of the resin in the coating layer (in terms of solid content; hereinafter, sometimes referred to as "amount of resin coating".). The surface roughness of a surface of the paper substrate, the surface being in contact with the coating layer, can be adjusted with the degree of fibrillation of the pulp constituting a paper layer having the relevant surface, as described above. The surface roughness of a surface of the coating layer, the surface being located opposite to the paper substrate, is smaller as the amount of resin coating is larger. The amount of resin coating is not particularly limited as long as the surface roughness of a surface of the coating layer, the surface being located opposite to the paper substrate, can be allowed to fall within the above range, and the amount is preferably 0.10 g/m² or more, more preferably 0.30 g/m² or more, further preferably 0.40 g/m² or more, still more preferably 0.50 g/m² or more, particularly preferably 1.00 g/m² or more in order to more effectively suppress generation of paper dust from the paper board. The amount of resin coating is preferably 1.90 g/m² or less, more preferably 1.80 g/m² or less, further preferably 1.60 g/m² or less, still more preferably 1.50 g/m² or less, in order to ensure the surface roughness at a level which enables the paper board to hardly slide. In other words, examples of the amount of resin coating include ranges of 0.10 to 1.90 g/m², 0.30 to 1.80 g/m², 0.40 to 1.60 g/m², 0.50 to 1.50 g/m², and 1.00 to 1.50 g/m² or more.

The amount of resin coating is measured by the following method.

First, the type of the resin is identified by surface analysis of the paper board for skin pack. The method for identifying the type of the resin is not particularly limited, and examples include a method with an iodide/starch reaction in a case where the resin is, for example, starch. Next, the thermoplastic resin layer and the paper substrate are removed with a polishing apparatus (for example, an apparatus manufactured by Sagawa Manufacturing, Inc.; a dimension of grinding stone of φ50.8 × 12.7 mm) so that a portion from a surface to a depth of 20 µm of the coating layer of the paper board for skin pack remains. Thereafter, the remaining coating-layer-attached paper piece after polishing is used to measure the content of the resin. The method for measuring the content of the resin is not particularly limited, and any method such as a known method can be adopted. For example, in a case where the resin is starch, the content of the resin can be measured by a biosensor method. More specifically, the amount of starch can be calculated by treating the coating-layer-attached paper piece with an amylase solution containing glucoamylase, filtrating the treatment liquid with a filter (for example, "Ekicrodisc13" manufactured by Pall Corporation Japan), and measuring the concentration of the starch in the resulting filtrate with a biosensor (for example, "BF-2" manufactured by Oji Scientific Instruments).

In a case where the paper strengthening agent and the resin in the coating layer are made of the same type of resin, for example, in a case where both the paper strengthening agent and the resin in the coating layer are starch-based resins, the amount of resin coating is measured by the following method.

First, in a case where the paper substrate is a combined sheet, the paper board for skin pack is polished with the polishing apparatus so that the coating layer and an adjacent paper layer to the coating layer remain. In a case where the paper substrate is a single sheet, the paper board for skin pack is polished with the polishing apparatus so that the coating layer and the single sheet remain. Thereafter, the amount of the starch-based resin contained in the remaining coating-layer-attached paper piece after polishing is measured.

Next, the paper board for skin pack is polished with the polishing apparatus so that a portion from a depth of 40 µm to 70 µm from the surface of the coating layer of the paper board for skin pack remains. Thereafter, the amount of the starch-based resin contained in the remaining paper piece after polishing is measured. An almost constant concentration of the starch-based resin relative to the pulp is here detected around the center of the paper piece. This starch-based resin corresponds to the starch-based resin internally added as the paper strengthening agent to the paper layer or the single sheet, and it can be said that this concentration of the starch-based resin is uniformly distributed in the entire paper layer or the entire single sheet. This is utilized to calculate the amount of the starch-based resin internally added as the paper strengthening agent to the paper layer or the single sheet of the coating-layer-attached paper piece. The amount of resin coating is calculated by subtracting "the amount of the starch-based resin internally added as the paper strengthening agent to the paper layer or the single sheet of the coating-layer-attached paper piece" from "the amount of the starch-based resin contained in the coating-layer-attached paper piece".

The coating layer may contain a component other than the resin as long as the effects of the present disclosure are not impaired. Examples of the component other than the resin include titanium oxide, kaolin, and calcium carbonate.

In a case where the coating layer contains titanium oxide, the coating layer also serves as a laser printing layer described below. In a case where the coating layer contains titanium oxide, the content of the titanium oxide in the coating layer has the same meaning as the content of the titanium oxide in a laser printing layer described below, and the same also applies to its preferred aspects.

### 1-3. Thermoplastic resin layer

The paper board for skin pack has a thermoplastic resin layer on one surface of the paper substrate. The thermoplastic resin layer serves to protect a surface of the paper board for skin pack from a contained article such as a food, and/or to protect such a contained article, from an external stimulus such as oxygen. For example, when a contained article is sandwiched and contained between the paper board for skin pack and a resin film, the thermoplastic resin layer serves to adhere with the resin film.

The thermoplastic resin layer may be a single layer of a thermoplastic resin, or a layered body including a thermoplastic resin, and is preferably a layered body including a barrier layer, in particular, preferably a layered body having a barrier layer between thermoplastic resin layers, as in "thermoplastic resin layer/barrier layer/thermoplastic resin layer". The thermoplastic resin layer may also be a laminated layer.

In a case where the thermoplastic resin layer is a layered body having a barrier layer, the barrier layer is not particularly limited as long as it can serve as a barrier against oxygen and/or water vapor, and it may be a layer having a single-layer structure, or a layer having a multi-layer structure of two or more layers.

The resin forming the barrier layer is specifically preferably one or more selected from the group consisting of a polyamide-based resin, polyvinyl alcohol (PVOH), an ethylene-vinyl alcohol copolymer (EVOH), and a polyvinylidene chloride resin, more preferably one or more selected from the group consisting of a polyamide-based resin, polyvinyl alcohol, and an ethylene-vinyl alcohol copolymer, further preferably an ethylene-vinyl alcohol copolymer. The polyamide-based resin is preferably an aromatic polyamide, more preferably polyamide MXD6. The barrier layer may also contain one or more other resins such as a biomass resin and a biodegradable resin.

Alternatively, the barrier layer may also be a metal layer.

The thickness of the barrier layer is not particularly limited, and it is preferably 1 to 100 µm, more preferably 3 to 50 µm, further preferably 5 to 30 µm.

The thermoplastic resin used in the thermoplastic resin layer is preferably one that can be laminated on the paper layer. Any one such as a known thermoplastic resin can be adopted as the thermoplastic resin.

Examples of such a known thermoplastic resin include polyester-based resins such as polyethylene terephthalate, polybutylene terephthalate, polyethylene naphthalate, polylactic acid, and polybutylene succinate; polyolefin-based resins such as polyvinyl chloride, polyvinylidene chloride, polybutene, polybutadiene, an ethylene-vinyl acetate copolymer, polyethylene, polypropylene, an ethylene-propylene copolymer, and polymethylpentene; polycarbonate; polyurethane; polyamide such as nylon 6; polyacrylonitrile; and poly(meth)acrylate.

Among these thermoplastic resins, the thermoplastic resin is preferably a polyolefin-based resin, more preferably one or more selected from the group consisting of polyethylene (PE) and polypropylene (PP), because extrusion lamination properties and barrier properties are excellent. PE is preferably one or more selected from the group consisting of low-density polyethylene (LDPE) and medium-density polyethylene (MDPE) because extrusion lamination properties are excellent.

The thickness of the entire thermoplastic resin layer is not particularly limited, and it is preferably 1 to 200 µm, more preferably 1 to 100 µm, further preferably 10 to 80 µm, still more preferably 20 to 50 µm.

Examples of a commercially available product of a resin layered body (layered film) suitably used in the thermoplastic resin layer include a three-layer film (trade name: Diamiron YF1966, film thickness 40 µm) of PE (polyethylene)/EVOH (ethylene-vinyl alcohol copolymer)/PP (polypropylene) manufactured by Mitsubishi Chemical Corporation, a three-layer film (trade name: ECO-B, film thickness 20 µm) of PP/PVOH (polyvinyl alcohol)/PP manufactured by Futamura Chemical Co., Ltd., a three-layer film (trade name: Heptax HP, film thickness 17 µm) of NY6 (nylon 6)/EVOH/NY6 manufactured by Gunze Ltd., and a three-layer film (trade name: Emblon M, film thickness 15 µm) of NY6/MXD6 (polyamide MXD6)/NY6 manufactured by Unitika Ltd.

### 1-4. Pressure-sensitive adhesive layer

In a case where a surface of the thermoplastic resin layer has adhesiveness, the thermoplastic resin layer may also be allowed to directly adhere to a surface of the paper substrate, but a pressure-sensitive adhesive layer may be located between the paper substrate and the thermoplastic resin layer in order to suppress peeling during production, transport, display, and the like of the skin pack package.

The pressure-sensitive adhesive constituting the pressure-sensitive adhesive layer is not particularly limited, and is preferably a resin-based pressure-sensitive adhesive suitable for dry lamination and wet lamination.

Specifically, aqueous, solvent-based, UV-based, and other pressure-sensitive adhesives can be used in the pressure-sensitive adhesive constituting the pressure-sensitive adhesive layer, and in particular, an aqueous pressure-sensitive adhesive is preferred. Such an aqueous pressure-sensitive adhesive is preferably at least one selected from the group consisting of an acrylic pressure-sensitive adhesive, a polyurethane-based pressure-sensitive adhesive, and an isocyanate-based pressure-sensitive adhesive, more preferably an acrylic pressure-sensitive adhesive.

The mass per unit area (in terms of solid content) of the pressure-sensitive adhesive layer is preferably 1 to 50 g/m², more preferably 5 to 20 g/m². The pressure-sensitive adhesive layer is preferably formed by coating a paper substrate surface or a thermoplastic resin layer surface with the pressure-sensitive adhesive so that the mass per unit area of the pressure-sensitive adhesive layer falls within the above range.

For such coating, a coating liquid containing the pressure-sensitive adhesive is preferably used, and a mixed coating liquid in which a curing agent is mixed with a coating liquid containing the pressure-sensitive adhesive is more preferably used.

The thickness of the pressure-sensitive adhesive layer is not particularly limited, and it is preferably 5 to 100 µm, more preferably 8 to 30 µm from the viewpoint of molding processability.

### 1-5. Other layers

The paper board for skin pack according to the present embodiment may include any other layer than the above as long as the effects of the present disclosure are not impaired. Examples of such other layer include an ink layer including an ink; and a laser printing layer that can be printed by ultraviolet laser printing.

### (Ink layer)

The ink layer is a layer formed by printing. The printing method is not particularly limited, and, for example, any method such as offset printing or ink-jet printing can be adopted.

The ink layer can be provided, for example, on a surface of the thermoplastic resin layer, the surface being located opposite to a surface of the thermoplastic resin layer, the surface being in contact with the paper substrate, between the thermoplastic resin layer and the paper substrate, and on the coating layer. In particular, the ink layer is preferably provided between the thermoplastic resin layer and the paper substrate. In a case where the ink layer is disposed between the thermoplastic resin layer and the paper substrate, it is preferable that the ink layer be provided so as to be adjacent to the paper substrate and the thermoplastic resin layer be provided on the ink layer with the pressure-sensitive adhesive layer being interposed.

In a case where the ink layer is provided on the coating layer, the ink layer may be provided at any position on the coating layer, but is preferably provided at a position which corresponds to one portion of the coating layer and which is not grounded when the skin pack package is placed on a slope from the viewpoint that the paper board for skin pack hardly slides.

### (Laser printing layer)

The laser printing layer is a printing layer containing titanium oxide. When the laser printing layer is irradiated with an ultraviolet laser, the color of the titanium oxide in the printing layer is changed from white to black, and characters, patterns, and/or the like are printed. This change in color of the titanium oxide is considered to occur by the change in ionic valence of the titanium oxide contained in the printing layer, from tetravalence to trivalence, and then the occurrence of oxygen defects.

The laser printing layer can be provided, for example, on a surface of the thermoplastic resin layer, the surface being located opposite to a surface of the thermoplastic resin layer, the surface being in contact with the paper substrate, between the thermoplastic resin layer and the paper substrate, and on the coating layer. Alternatively, the coating layer or the paper substrate may also be allowed to contain titanium oxide, thereby also serving as the laser printing layer.

In a case where the laser printing layer is provided on the coating layer, the laser printing layer is preferably provided at a position which corresponds to one portion of the coating layer and which is not grounded when the skin pack package is placed on a slope, in order to ensure that the paper board for skin pack hardly slides.

The method for forming the laser printing layer is not particularly limited, may be a coating method or a lamination method, and is preferably a coating method because the laser printing layer can be provided only at a desired location and production is easily made.

The content of titanium oxide in the laser printing layer is usually 0.1 g/m² or more, preferably 0.2 g/m² or more, more preferably 0.3 g/m² or more, further preferably 0.4 g/m² or more from the viewpoint of providing a sufficient printing density, and is preferably 10 g/m² or less, more preferably 7.5 g/m² or less, further preferably 5 g/m² or less, still more preferably 3.5 g/m² or less from the viewpoint of providing a printing spot excellent in printing clarity, from the viewpoint of suppressing an increase in cost due to saturation of the printing density and thus inclusion of titanium oxide in an amount equal to or more than the required amount, and from the viewpoint of suppressing the amount of smoking during irradiation with an ultraviolet laser (during printing).

Although the occurrence of smoking which is considered to be due to scattering of titanium oxide during irradiation with an ultraviolet laser has the risk of causing dropping of titanium oxide changed in color, from the laser printing layer, and such dropping of titanium oxide changed in color, from the laser printing layer, also tends to cause deterioration in printing clarity, such smoking tends to be suppressed by setting the content of titanium oxide in the laser printing layer to the above upper limit or less.

A case where the laser printing layer is provided between the thermoplastic resin layer and the paper substrate is further effective because the thermoplastic resin layer functions as a protection layer, to not only suppress smoking due to irradiation with an ultraviolet laser, but also suppress desorption of titanium oxide due to the smoking, resulting in a tendency to provide a print image having a high printing concentration and having excellent printing clarity per point.

### 1-6. Method for producing paper board for skin pack

The method for producing the paper board for skin pack according to the present embodiment is not particularly limited, any method such as a known method or a method according thereto can be adopted. One example of the following production method is shown.

### (Production of paper substrate)

First, a paper layer having a single-layer or multi-layer structure is obtained by single-layer papermaking or multi-layer papermaking from a paper material including pulp. The paper layer obtained can be in the form of a single sheet to serve as the paper substrate. The paper layer can also be bonded, if necessary, with an adhesive layer and thus can be in the form of a combined sheet to serve as the paper substrate.

When the paper material is prepared, an internal additive may also be added to pulp. Examples of the internal additive include a sizing agent, a loading material, a paper strengthening agent, a yield improver, a pH adjuster, a freeness-improving agent, a waterproofing agent, a softener, an antistatic agent, a defoamer, a slime control agent, a dye, and a pigment. Among these, the internal additive is preferably one or more selected from the group consisting of a rosin-based sizing agent, a paper strengthening agent, and aluminum sulfate.

The amount of the rosin-based sizing agent added to 100 parts by mass of the pulp in preparation of the paper material is preferably 0.05 to 0.50 parts by mass.

The amount of the paper strengthening agent added to 100 parts by mass of the pulp in preparation of the paper material is not particularly limited as long as the content of the paper strengthening agent based on the total amount of the paper substrate falls within the above range, and the amount is preferably 0.05 to 5.00 parts by mass, more preferably 0.10 to 4.00 parts by mass, further preferably 0.40 to 3.00 parts by mass, still more preferably 0.80 to 2.00 parts by mass, particularly preferably 1.00 to 1.80 parts by mass.

The amount of the aluminum sulfate added to 100 parts by mass of the pulp in preparation of the paper material is preferably 0.05 to 0.50 parts by mass.

Any wet paper machine can be appropriately selected and used for papermaking from the paper material. More specifically, examples of the paper machine include a twin-wire paper machine, a Fourdrinier paper machine, a gap former-type paper machine, a cylinder paper machine, and a tanmo paper machine.

Examples of papermaking from the paper material include a method including casting the paper material on a wire or the like and performing dewatering to provide wet paper, if necessary, stacking plural sheets of paper, and pressing and drying such single-layer or multi-layer wet paper. Here, in a case where plural sheets of paper are not stacked, single-layer base paper is obtained, and in a case where plural sheets of paper are stacked, multi-layer base paper is obtained. In a case where such multi-layer base paper is obtained, the above paper strengthening agent may be applied between layers during papermaking and such layers may be combined, in order to increase the interlayer strength.

In a case where the paper strengthening agent is applied, the mass per unit area (in terms of solid content) of the paper strengthening agent is not particularly limited as long as the content of the paper strengthening agent based on the total amount of the paper substrate falls within the above range, and the mass is preferably 0.10 to 5.00 g/m², more preferably 0.40 to 2.00 g/m².

The paper produced with the paper machine is preferably, for example, conveyed by felt and dried by a dryer. A multistage cylinder dryer may also be used as a pre-dryer before such drying by a dryer.

The resulting paper may also be surface-treated by a calender to make the thickness and the profile uniform. Any calender treatment machine can be appropriately selected and used for such a calender treatment.

In a case where the paper substrate is a single sheet, the paper board for skin pack can be obtained by providing the coating layer and the thermoplastic resin layer with the resulting paper layer as the paper substrate.

In a case where the paper substrate is a combined sheet, the method for producing the paper board for skin pack after the paper layer A and the paper layer B are obtained is not particularly limited, and examples thereof include a method 1 including bonding the paper layer A and the paper layer B with an adhesive layer being interposed, to obtain the paper substrate, and then providing the coating layer and the thermoplastic resin layer; a method 2 including providing the coating layer on the paper layer B, providing an adhesive layer and the paper layer A on a surface of the paper layer B, the surface being located opposite to a surface of the paper layer B, the surface being in contact with the coating layer, and further providing the thermoplastic resin layer on the paper layer A; and a method 3 including providing the thermoplastic resin layer on the paper layer A, providing an adhesive layer and the paper layer B on a surface of the paper layer A, the surface being located opposite to a surface of the paper layer A, the surface being in contact with the thermoplastic resin layer, and further providing the coating layer on the paper layer B.

Alternatively, a combined sheet being the paper substrate can also be obtained by coating a surface of any of the paper layers with an adhesive coating liquid containing an aqueous solution or an emulsion in which the resin contained in the above adhesive layer is dissolved or dispersed in water, if necessary, removing some or all moisture, and attaching the other of the paper layers to the surface coated. In this case, such coating with the adhesive coating liquid can be performed with a coater such as a blade coater, an air knife coater, a roll coater, a bar coater, a gravure coater, a rod blade coater, a lip coater, a die coater, or a curtain coater. Such removal of moisture is preferably performed by, for example, drying by hot air, or drying by irradiation with infrared light.

A printing step of performing printing on a paper layer surface or a printing-layer-attaching step of providing a printing layer may be, if necessary, performed before or after the attaching step of each of the paper layers.

### (Formation of coating layer)

The coating layer can be formed by coating one surface of the paper substrate with a coating liquid in which the resin is dissolved or suspended in a solvent, and removing the solvent of the coating liquid by drying. The coating liquid is preferably one in which the resin is dissolved in the solvent, more preferably one in which the water-soluble resin is dissolved in water because the surface roughness of the paper substrate is more easily adjusted. The same method as in coating with or drying of the adhesive coating liquid described above can be adopted for coating with and drying of the coating liquid.

### (Formation of thermoplastic resin layer)

The thermoplastic resin layer may directly adhere to the paper substrate, or may adhere to the paper substrate with the pressure-sensitive adhesive layer being interposed. In the latter case, it is preferable to coat a paper substrate surface with a coating liquid containing the pressure-sensitive adhesive in advance, and layer the thermoplastic resin layer on a surface of the paper substrate with the pressure-sensitive adhesive layer being interposed. A film as the thermoplastic resin layer may also be here bonded with the paper substrate.

### (Processing)

The paper board for skin pack, obtained as above, may also be cut out to an appropriate dimension in consideration of the size and the shape of a contained article, transport, and suitability to showing. Such cutting out is preferably made by punching processing from the viewpoint of efficiently providing a paper board for skin pack, having the same shape.

Such punching processing is preferably performed with a high-speed automatic punching machine, a plate punching machine, or a rotary punching machine, and is more preferably performed with a high-speed automatic punching machine. Such punching processing can be performed with a high-speed automatic punching machine or a plate punching machine, thereby easily and efficiently providing a paper board for skin pack, having any shape such as a quadrangle, a rounded-corner quadrangle, and an oval shape.

### 2. Skin pack package

A second embodiment of the present disclosure relates to a skin pack package including the above paper board for skin pack, a contained article, and a resin film, wherein the contained article is contained between the thermoplastic resin layer of the paper board for skin pack and the resin film. The skin pack package according to the present embodiment is preferably one in which the contained article is hermetically received.

The resin film can be appropriately selected depending on the type, the shape, and the like of the contained article, and is preferably one having excellent adhesiveness and also having releasability in extraction of the contained article because the resin film is required to adhere with the thermoplastic resin layer of the paper board for skin pack and thus contain the contained article. Accordingly, the resin film is preferably one constituted from the above-described thermoplastic resin used in the thermoplastic resin layer, and is more preferably one having a barrier layer in a case where the content is a food or the like. The barrier layer used here is suitably the barrier layer used in the above-described thermoplastic resin layer.

In a case where the skin pack package according to the present embodiment is preserved, displayed, and/or the like with being suspended, the skin pack package is provided with a suspension part. The suspension part is not particularly limited as long as it can allow the skin pack package to be suspended by a suspension rod, and may be, for example, a through-hole which is opened in the skin pack package so that the suspension rod can pass through the through-hole, a part which is formed by cutting one portion of the skin pack package into a hook shape so that the part can be put on the suspension rod, or a suspension tool which is mounted to the skin pack package, such as a hook or an adhesive disc.

The contained article is not particularly limited, and examples include foods and daily goods. The skin pack package can hermetically contain the contained article, and is suitable for containing foods, for example, fresh foods such as vegetables, dressed meat, and fresh fish; and processed foods thereof, as the contained article.

### EXAMPLES

Hereinafter, the present disclosure is further specifically described with reference to Examples and Comparative Examples. The materials, the amounts of use, the proportions, the treatment contents, the treatment procedures, and the like shown in the following Examples can be appropriately changed without departing from the gist of the present disclosure. Accordingly, the scope of the present disclosure should not be interpreted in a limited manner by specific examples shown below.

### [Production of paper layer A1]

A pulp slurry having a Canadian standard freeness (CSF) of 490 mL was obtained by mixing 50 parts by mass of leaf bleached kraft pulp (LBKP) and 50 parts by mass of needle bleached kraft pulp (NBKP). A double disc refiner was used for beating. To the pulp slurry obtained were added 0.80 parts by mass (in terms of solid content) of aluminum sulfate (manufactured by Asahi Chemical Co., Ltd.), 0.1 parts by mass (in terms of solid content)of a polyacrylamide-based paper strengthening agent ("Polystron 1430" manufactured by Arakawa Chemical Industries, Ltd.), and 0.12 parts by mass (in terms of solid content) of an internal rosin sizing agent ("Sizepine N-817" manufactured by Arakawa Chemical Industries, Ltd.) based on 100 parts by mass (in terms of solid content) of the pulp, and thus a paper material was prepared. The paper material was used for papermaking performed with a tanmo paper machine for five-layer papermaking, in which all the set basis weights of five layers (front layer, front lower-layer, middle layer, rear lower-layer, and rear layer) were the equal amounts and the set basis weight was 450 g/m², and thus a paper layer A1 was obtained.

### [Production of paper layer A2]

A paper layer A2 was obtained in the same manner as in the paper layer A1 except that the amount of the paper strengthening agent added based on 100 parts by mass (in terms of solid content) of the pulp, in preparation of the paper material, was changed to 0.50 parts by mass.

### [Production of paper layer A3]

A paper layer A3 was obtained in the same manner as in the paper layer A1 except that the amount of the paper strengthening agent added based on 100 parts by mass (in terms of solid content) of the pulp, in preparation of the paper material, was changed to 1.50 parts by mass.

### [Production of paper layer A4]

A paper layer A4 was obtained in the same manner as in the paper layer A1 except that the amount of the paper strengthening agent added based on 100 parts by mass (in terms of solid content) of the pulp, in preparation of the paper material, was changed to 2.00 parts by mass.

### [Production of paper layer A5]

A paper layer A5 was obtained in the same manner as in the paper layer A1 except that the degree of beating was adjusted so that the CSF was 560 mL in preparation of the pulp slurry, and the amount of the paper strengthening agent added based on 100 parts by mass (in terms of solid content) of the pulp, in preparation of the paper material, was changed to 0.40 parts by mass.

### [Production of paper layer A6]

A paper layer A6 was obtained in the same manner as in the paper layer A1 except that the degree of beating was adjusted so that the CSF was 620 mL in preparation of the pulp slurry, and the amount of the paper strengthening agent added based on 100 parts by mass (in terms of solid content) of the pulp, in preparation of the paper material, was changed to 0.80 parts by mass.

### [Production of paper layer A7]

A paper layer A7 was obtained in the same manner as in the paper layer A1 except that the degree of beating was adjusted so that the CSF was 620 mL in preparation of the pulp slurry, and the amount of the paper strengthening agent added based on 100 parts by mass (in terms of solid content) of the pulp, in preparation of the paper material, was changed to 1.80 parts by mass.

### [Production of coating-layer-attached paper layer A8]

A paper layer was produced in the same manner as in the paper layer A7 except that the amount of the paper material and the pressing pressure during papermaking were adjusted so that the basis weight was 520 g/m². A surface of the paper layer was coated by a bar coater with starch ("GRS-T110" manufactured by Oji Cornstarch Co., Ltd.) adjusted to an aqueous 10% by mass solution so that the amount of coating in terms of solid content was 0.50 g/m², and thus a coating-layer-attached paper layer A8 was obtained.

### [Production of paper layer A9]

A paper layer A9 was obtained in the same manner as in the paper layer A1 except that the paper strengthening agent was not added to the pulp slurry in preparation of the paper material.

### [Production of paper layer A10]

A paper layer A10 was obtained in the same manner as in the paper layer A1 except that the degree of beating was adjusted so that the CSF was 380 mL in preparation of the pulp slurry.

### [Production of coating-layer-attached paper layer B1]

A pulp slurry having a Canadian standard freeness (CSF) of 490 mL was obtained by mixing 50 parts by mass of leaf bleached kraft pulp (LBKP) and 50 parts by mass of needle bleached kraft pulp (NBKP). A double disc refiner was used for beating. To the pulp slurry obtained were added 0.80 parts by mass (in terms of solid content) of aluminum sulfate (manufactured by Asahi Chemical Co., Ltd.), 0.1 parts by mass (in terms of solid content)of a polyacrylamide-based paper strengthening agent ("Polystron 1430" manufactured by Arakawa Chemical Industries, Ltd.), and 0.12 parts by mass (in terms of solid content) of an internal rosin sizing agent ("Sizepine N-817" manufactured by Arakawa Chemical Industries, Ltd.) based on 100 parts by mass (in terms of solid content) of the pulp, and thus a paper material was prepared. The paper material was used for papermaking performed with a twin-wire paper machine, in which the set basis weight was 70 g/m², and thus a paper layer was produced. A surface of the paper layer was coated by a bar coater with oxidized starch ("Ace A" manufactured by Oji Cornstarch Co., Ltd.) adjusted to an aqueous 10% by mass solution so that the amount of coating in terms of solid content was 0.50 g/m², and thus a coating-layer-attached paper layer B1 was obtained.

### [Production of coating-layer-attached paper layer B2]

A coating-layer-attached paper layer B2 was obtained in the same manner as in the coating-layer-attached paper layer B1 except that the amount of coating of the surface of the paper substrate with the oxidized starch, in terms of solid content, was changed to 1.00 g/m².

### [Production of coating-layer-attached paper layer B3]

A coating-layer-attached paper layer B3 was obtained in the same manner as in the coating-layer-attached paper layer B1 except that the amount of coating of the surface of the paper substrate with the oxidized starch, in terms of solid content, was changed to 1.50 g/m².

### [Production of coating-layer-attached paper layer B4]

A coating-layer-attached paper layer B4 was obtained in the same manner as in the coating-layer-attached paper layer B1 except that the amount of the paper strengthening agent added based on 100 parts by mass (in terms of solid content) of the pulp, in preparation of the paper material, was changed to 0.50 parts by mass.

### [Production of coating-layer-attached paper layer B5]

A coating-layer-attached paper layer B5 was obtained in the same manner as in the coating-layer-attached paper layer B1 except that the amount of the paper strengthening agent added based on 100 parts by mass (in terms of solid content) of the pulp, in preparation of the paper material, was changed to 1.50 parts by mass.

### [Production of coating-layer-attached paper layer B6]

A coating-layer-attached paper layer B6 was obtained in the same manner as in the coating-layer-attached paper layer B1 except that the amount of the paper strengthening agent added based on 100 parts by mass (in terms of solid content) of the pulp, in preparation of the paper material, was changed to 2.00 parts by mass.

### [Production of coating-layer-attached paper layer B7]

A coating-layer-attached paper layer B7 was obtained in the same manner as in the coating-layer-attached paper layer B1 except that the degree of beating was adjusted so that the CSF was 560 mL in preparation of the pulp slurry, and the amount of the paper strengthening agent added based on 100 parts by mass (in terms of solid content) of the pulp, in preparation of the paper material, was changed to 0.40 parts by mass.

### [Production of coating-layer-attached paper layer B8]

A coating-layer-attached paper layer B8 was obtained in the same manner as in the coating-layer-attached paper layer B1 except that the degree of beating was adjusted so that the CSF was 620 mL in preparation of the pulp slurry, and the amount of the paper strengthening agent added based on 100 parts by mass (in terms of solid content) of the pulp, in preparation of the paper material, was changed to 0.80 parts by mass.

### [Production of coating-layer-attached paper layer B9]

A coating-layer-attached paper layer B9 was obtained in the same manner as in the coating-layer-attached paper layer B1 except that the degree of beating was adjusted so that the CSF was 620 mL in preparation of the pulp slurry, and the amount of the paper strengthening agent added based on 100 parts by mass (in terms of solid content) of the pulp, in preparation of the paper material, was changed to 1.80 parts by mass.

### [Production of paper layer B10]

A paper layer B10 was obtained in the same manner as in the coating-layer-attached paper layer B4 except that the surface of the paper substrate was not coated with the oxidized starch.

### [Production of paper layer B11]

A paper layer B11 was obtained in the same manner as in the coating-layer-attached paper layer B8 except that the surface of the paper substrate was not coated with the oxidized starch.

### [Production of coating-layer-attached paper layer B12]

A coating-layer-attached paper layer B12 was obtained in the same manner as in the coating-layer-attached paper layer B1 except that the paper strengthening agent was not added to the pulp slurry in preparation of the paper material.

### [Production of coating-layer-attached paper layer B13]

A coating-layer-attached paper layer B13 was obtained in the same manner as in the coating-layer-attached paper layer B1 except that the amount of coating of the surface of the paper substrate with the oxidized starch, in terms of solid content, was changed to 2.00 g/m².

### [Production of coating-layer-attached paper layer B14]

A coating-layer-attached paper layer B14 was obtained in the same manner as in the coating-layer-attached paper layer B1 except that the degree of beating was adjusted so that the CSF was 380 mL in preparation of the pulp slurry.

### [Example 1]

One surface of the paper layer A1 was coated by melt-extrusion with MDPE ("8010" manufactured by ENEOS NUC Corporation) so that the dry film thickness was 20 µm, and the paper layer B1 was bonded with the surface coated and thus a paper substrate of two-layer constitution was obtained. Thereafter, the paper layer A1 was coated with a coating liquid prepared by mixing 100 parts by mass of an aqueous acrylic pressure-sensitive adhesive ("EM-575" manufactured by ARAKAWA TORYO KOGYO CO., LTD.) and 3 parts by mass of a curing agent ("EM-550K" manufactured by ARAKAWA TORYO KOGYO CO., LTD.) so that the amount of coating after heat drying was 10 g/m², and thus a pressure-sensitive adhesive layer was formed. A three-layer film of PE/EVOH/PP ("Diamiron YF1966" manufactured by Mitsubishi Chemical Corporation, thickness 40 µm) was bonded onto the pressure-sensitive adhesive layer by dry lamination so that the PP layer and the pressure-sensitive adhesive layer were adjacent, and thus a paper board for skin pack was obtained.

### <Examples 2 to 9, and Comparative Examples 1 to 5>

Each paper board for skin pack was produced in the same manner as in Example 1 except that the paper layer was changed to one shown in Table 1.

### <Example 10>

A surface of the paper layer A8, the surface being located opposite to a surface of the paper layer A8, the surface being coated with starch, was coated with a coating liquid prepared by mixing 100 parts by mass of an aqueous acrylic pressure-sensitive adhesive ("EM-575" manufactured by ARAKAWA TORYO KOGYO CO., LTD.) and 3 parts by mass of a curing agent ("EM-550K" manufactured by ARAKAWA TORYO KOGYO CO., LTD.) so that the amount of coating after heat drying was 10 g/m², and thus a pressure-sensitive adhesive layer was formed. A film of three-layer constitution of PE/EVOH/PP ("Diamiron YF1966" manufactured by Mitsubishi Chemical Corporation, thickness 40 µm) was bonded onto the pressure-sensitive adhesive layer by dry lamination so that the PP layer and the pressure-sensitive adhesive layer were adjacent, and thus a paper board for skin pack was obtained.

Measurement and evaluation of physical properties of the paper substrate and the paper board for skin pack were carried out by the following methods. The results are shown in Table 1 and Table 2.

### [Basis weight]

The basis weight was measured with a high-resolution balance ("Sartorius Cubis MSU524S" manufactured by Sartorius Japan K.K.), according to JIS P 8124:2011 ("Paper and Board-Determination of basis weight", published on March 22, 2011).

### [Thickness]

The thickness was measured at ten points of a sample with digital thickness gauge ("No.132" manufactured by Toyo Seiki Seisaku-sho, Ltd.), according to JIS P 8118:2014 ("Paper and Board-Determination of thickness, density and specific volume", published on November 20, 2014), and the arithmetic mean value was defined as the paper thickness.

### [Surface roughness]

A backside of the paper board for skin pack was measured at a magnification of 12x with a 3D shape measuring machine ("VR3200" manufactured by Keyence Corporation). The entire region photographed, in the image obtained, was defined as the reference surface, wave removal at a wavelength of 5 mm or more was made with analysis software ("VR3000" manufactured by Keyence Corporation), the arithmetic mean height Sa was measured according to ISO 25178-2:2021 ("Geometrical Product Specifications (GPS)-Surface texture-Part 2: Terms, Definitions, and Surface texture parameters", published on April 2012), and then was defined as the surface roughness of a surface of the coating layer, the surface being located opposite to the paper substrate.

### [Specific tensile strength of paper board for skin pack]

The specific tensile strength in the longitudinal direction and the specific tensile strength in the lateral direction of the paper board for skin pack were measured with a horizontal tensile tester ("CODE SE-06" manufactured by L&W), according to JIS P 8113:2006 ("Paper and Board-Determination of tensile properties-Part 2: Constant rate of elongation method, published on December 20, 2006), and the geometric mean value thereof was calculated.

### [Degree of fibrillation of pulp]

The paper substrate of the paper board for skin pack was released by hand and cut to a 4-cm square, and the resultant was immersed in ion-exchange water and adjusted to a solid content concentration of 2% by mass, and then immersed for 24 hours. Thereafter, a standard defibrator (manufactured by Kumagai Riki Kogyo, Co., Ltd.) was used to disintegrate the pulp to a fiber form by a disintegration treatment performed for 20 minutes. In a case where non-disintegrated pulp was present, the disintegration treatment was again performed for 20 minutes to disintegrate the pulp to a fiber form. Such disintegration was here performed according to JIS P 8220-2:2012 ("Pulps-Disintegration -Part 2: Disintegration of Mechanical Pulps (20°C)", published on April 20, 2012).

A fiber length analyzer ("New Valmet FS5" manufactured by Valmet Corporation, equipped with UHD base unit) was used to measure the degree of fibrillation of a sample obtained by according to ISO 16065-2:2014 ("Pulps-Determination of fiber length by automated optical analysis-Part 2: Polarized light method", published on January 15, 2007). The proportion of a projected portion of a fuzzy fine fiber on a fiber surface, in the entire fiber projected, was calculated by expression (1), and defined as the degree of fibrillation.

### [Evaluation of suppression of generation of paper dust from paper board for skin pack]

An automatic plate punching machine was used for punching to obtain rectangular paper pieces of 280 mm length (MD) × 180 mm width (CD) from 100 paper boards for skin pack (550 mm length × 800 mm width) in a condition of 6000 pieces/hour. Whether or not paper dust attached to the thermoplastic resin layer was present was confirmed for ten paper boards for skin pack, punched at last, among the above paper boards for skin pack, and was evaluated according to the following criteria. AA to B were rated as good, and C was rated as poor.

### Evaluation criteria

AA: Zero paper boards for skin pack, in which paper dust was attached to the thermoplastic resin layer
A: One paper board for skin pack, in which paper dust was attached to the thermoplastic resin layer
B: Two paper boards for skin pack, in which paper dust was attached to the thermoplastic resin layer
C: Three or more paper boards for skin pack, in which paper dust was attached to the thermoplastic resin layer

### [Evaluation of sliding resistance of paper board for skin pack]

A 150-g weight (rectangular body of 150 mm length × 100 mm width × 10 mm height) was put on the central portion of the paper board for skin pack (rectangular shape of 280 mm length × 180 mm width), a barrier film ("Cryovac" manufactured by Sealed Air Corp) was used to perform skin pack packaging, and thus a skin pack package was produced.

A SUS plate (600 mm length × 300 mm width) was obliquely fixed on a horizontal hard surface, and a barrier film (manufactured by Sealed Air Corp; 550 mm length × 250 mm width) was bonded to an upper surface of the SUS plate. Three types of slopes were here each formed so that the angle of inclination of the SUS plate was 20 degrees, 23 degrees, or 26 degrees. The skin pack package was gently put on such each slope so that the barrier film and the paper board for skin pack were contacted with each other, and whether or not the skin pack package, when unhanded, slid down was observed. Evaluation was performed based on the angle of the slope at which the skin pack package slid down, according to the following criteria. AA to B were rated as good, and C was rated as poor.

### Evaluation criteria

AA: the skin pack package did not slide down even when put on the slope at an angle of inclination of 26 degrees
A: the skin pack package slid down from the slope at an angle of inclination of 26 degrees, but did not slide down from the slope at an angle of inclination of 23 degrees
B: the skin pack package slid down from the slope at an angle of inclination of 23 degrees, but did not slide down from the slope at an angle of inclination of 20 degrees
C: the skin pack package slid down from the slope at an angle of inclination of 20 degrees

### [Evaluation of warpage of paper board for skin pack during skin pack packaging]

A 150-g weight (rectangular body of 150 mm length × 100 mm width × 10 mm height) was put on the central portion of the paper board for skin pack (rectangular shape of 280 mm length × 180 mm width), and a barrier film ("Cryovac" manufactured by Sealed Air Corp) was used to perform skin pack packaging. The change in degree of warpage of the paper board for skin pack before and after skin pack packaging was evaluated by the following method.

First, the paper board was flatly placed on a horizontal hard surface, and floating at each of four corners of the paper board was measured and the average value was calculated. The warpage of the paper board before skin pack packaging and the warpage of the paper board after skin pack packaging were respectively measured, and the difference therebetween was evaluated according to the following criteria. AA to B were rated as good, and C was rated as poor.

### Evaluation criteria

AA: a difference in warpage of the paper board for skin pack before and after skin pack packaging, of less than 1 mm
A: a difference in warpage of the paper board for skin pack before and after skin pack packaging, of 1 mm or more and less than 3 mm
B: a difference in warpage of the paper board for skin pack before and after skin pack packaging, of 3 mm or more and less than 6 mm
C: a difference in warpage of the paper board for skin pack before and after skin pack packaging, of 6 mm or more

### [Evaluation of drop impact resistance of paper board for skin pack]

A 150-g weight (rectangular body of 150 mm length × 100 mm width × 10 mm height) was fixed to the central portion of the paper board for skin pack (rectangular shape of 280 mm length × 180 mm width), with a gummed cloth tape, and thus an evaluation sample was produced.

The evaluation sample was disposed at a position with a height of 500 mm from a vinyl chloride floor so that a shorter side would first contact the floor when the sample was allowed to drop. Thereafter, the evaluation sample was allowed to freely fall vertically onto the vinyl chloride floor, and the presence or absence of any fold formed in the paper board for skin pack was visually observed. This drop impact test was repeatedly performed, and the number of test times until any fold was formed in the evaluation sample was counted. The above operation was performed for five such evaluation samples, and the average number of test times until any fold was formed in the paper board for skin pack (rounded off at the second decimal place) was calculated and evaluated according to the following criteria. AA to B were rated as good, and C was rated as poor.

### Evaluation criteria

AA: an average number of test times until any fold was formed in the paper board for skin pack, of 5.0 or more
A: an average number of test times until any fold was formed in the paper board for skin pack, of 3.0 or more and less than 5.0
B: an average number of test times until any fold was formed in the paper board for skin pack, of 2.0 or more and less than 3.0
C: an average number of test times until any fold was formed in the paper board for skin pack, of less than 2.0

### [Table 1]

**Table 1**

| | Paper substrate | | | | | | | Coating layer |
|---|---|---|---|---|---|---|---|---|
| | Paper layer A | | | Paper layer B | | | Content of paper strengthening agent | Amount of resin |
| | Type | Basis weight^{*2} | CSF | Type | Basis weight^{*2} | CSF | | |
| | | (g/m²) | (mL) | | (g/m²) | (mL) | (% by mass) | (g/m²) |
| Example 1 | A1 | 450 | 490 | B1^{*1} | 70 | 490 | 0.10 | 0.50 |
| Example 2 | A1 | 450 | 490 | B2^{*1} | 70 | 490 | 0.10 | 1.00 |
| Example 3 | A1 | 450 | 490 | B3^{*1} | 70 | 490 | 0.10 | 1.50 |
| Example 4 | A2 | 450 | 490 | B4^{*1} | 70 | 490 | 0.50 | 0.50 |
| Example 5 | A3 | 450 | 490 | B5^{*1} | 70 | 490 | 1.50 | 0.50 |
| Example 6 | A4 | 450 | 490 | B6^{*1} | 70 | 490 | 2.00 | 0.50 |
| Example 7 | A5 | 450 | 560 | B7^{*1} | 70 | 560 | 0.40 | 0.50 |
| Example 8 | A6 | 450 | 620 | B8^{*1} | 70 | 620 | 0.80 | 0.50 |
| Example 9 | A7 | 450 | 620 | B9^{*1} | 70 | 620 | 1.80 | 0.50 |
| Example 10 | A8^{*1} | 520 | 620 | - | - | - | 1.80 | 0.50 |
| Comparative Example 1 | A2 | 450 | 490 | B10 | 70 | 490 | 0.50 | - |
| Comparative Example 2 | A6 | 450 | 620 | B11 | 70 | 620 | 0.80 | - |
| Comparative Example 3 | A9 | 450 | 490 | B12^{*1} | 70 | 490 | - | 0.50 |
| Comparative Example 4 | A1 | 450 | 490 | B13^{*1} | 70 | 490 | 0.10 | 2.00 |
| Comparative Example 5 | A10 | 450 | 380 | B14^{*1} | 70 | 380 | 0.10 | 0.50 |

| | | | | | | | | |
|---|---|---|---|---|---|---|---|---|
| *1: Coating-layer-attached paper layer *2: Basis weight of paper layer excluding coating layer in case of coating-layer-attached paper layer | | | | | | | | |

### [Table 2]

**Table 2**

| | Properties | | | | | Evaluation | | | |
|---|---|---|---|---|---|---|---|---|---|
| | Basis weight | Thickness | Geometric mean of specific tensile strength | Surface roughness of surface of coating layer, this surface being located opposite to paper substrate | Degree of fibrillation | Occurrence of paper dust | Sliding resistance | Warpage resistance during packaging | Drop impact resistance |
| | (g/m²) | (µm) | (Nm/g) | (µm) | (%) | | | | |
| Example 1 | 587 | 650 | 37.2 | 3.32 | 0.7 | B | A | B | AA |
| Example 2 | 587 | 655 | 38.9 | 2.57 | 0.7 | A | B | B | AA |
| Example 3 | 588 | 660 | 40 | 1.83 | 0.7 | AA | B | B | AA |
| Example 4 | 587 | 650 | 41.8 | 3.33 | 0.7 | A | A | A | AA |
| Example 5 | 587 | 650 | 51.1 | 3.34 | 0.7 | AA | A | AA | A |
| Example 6 | 587 | 650 | 55.2 | 3.33 | 0.7 | AA | A | AA | B |
| Example 7 | 587 | 650 | 37.3 | 3.52 | 0.4 | A | A | B | AA |
| Example 8 | 587 | 650 | 37.5 | 3.71 | 0.2 | AA | AA | B | AA |
| Example 9 | 587 | 650 | 47.9 | 3.76 | 0.2 | AA | AA | AA | AA |
| Example 10 | 568 | 630 | 47.4 | 3.75 | 0.2 | AA | AA | AA | AA |
| Comparative Example 1 | 586 | 645 | 40.3 | 4.42 | 0.7 | C | AA | B | AA |
| Comparative Example 2 | 586 | 645 | 37.1 | 4.72 | 0.2 | C | AA | B | AA |
| Comparative Example 3 | 587 | 650 | 35.4 | 3.39 | 0.7 | B | A | C | AA |
| Comparative Example 4 | 589 | 665 | 41.2 | 0.82 | 0.7 | AA | C | B | AA |
| Comparative Example 5 | 587 | 650 | 51.4 | 2.98 | 1.3 | C | B | AA | A |

### REFERENCE SIGN LIST

10 paper board for skin pack
11 thermoplastic resin layer
13 paper substrate
15 coating layer
20 fiber
21 fibril
23 external fibril

## Claims

1. A paper board for skin pack, comprising a thermoplastic resin layer, a paper substrate, and a coating layer in the listed order, wherein
a degree of fibrillation of pulp in the paper substrate is 1.0% or less,
the coating layer contains a resin,
a surface roughness of a surface of the coating layer, the surface being located opposite to the paper substrate, is 1.50 to 4.20 µm, and
a geometric mean of a specific tensile strength in a longitudinal direction and a specific tensile strength in a lateral direction of the paper board for skin pack is 37.0 N·m/g or more.

2. The paper board for skin pack according to claim 1, wherein a mass per unit area of the resin in the coating layer is 0.10 to 1.90 g/m².

3. The paper board for skin pack according to claim 1, wherein the resin contained in the coating layer contains one or more selected from the group consisting of a water-soluble resin and a water-suspendable resin.

4. The paper board for skin pack according to claim 3, wherein the water-soluble resin contains one or more selected from the group consisting of oxidized starch, cationized starch, polyvinyl alcohol, and polyacrylamide.

5. The paper board for skin pack according to claim 1, wherein the geometric mean of a specific tensile strength in a longitudinal direction and a specific tensile strength in a lateral direction of the paper board for skin pack is 53.0 N·m/g or less.

6. The paper board for skin pack according to claim 1, wherein the paper substrate contains 0.10% by mass or more of a paper strengthening agent based on a total amount of the paper substrate.

7. The paper board for skin pack according to claim 1, wherein the paper substrate is a combined sheet having a paper layer A, an adhesive layer and a paper layer B in the listed order, or a single sheet.

8. A skin pack package comprising the paper board for skin pack according to any one of claims 1 to 7, a contained article, and a resin film, wherein
the contained article is contained between the thermoplastic resin layer of the paper board for skin pack and the resin film.
